# EUROPEAN PATENT APPLICATION

(11) **EP 2 479 693 A1**
(43) Date of publication of application: **25.07.2012**
(21) Application number: 11290040.2
(22) Date of filing: 18.01.2011
(51) Int. Cl.: G06F 19/00

(54) **Method of managing health information**

(71) Applicant: Alcatel-Lucent Deutschland AG, 70435 Stuttgart (DE)
(72) Inventor: Grob-Lipski, Heldrun, 72181 Starzach (DE); Schefczik, Peter, 91056 Erlangen (DE); Roessler, Horst, 70794 Filderstadt (DE)
(74) Representative: Wetzel, Emmanuelle

(57) **Abstract**

Method of managing health information, the method comprising maintaining a personal health record for an individual by a provider and authorizing access to the personal health record in response to a medical emergency, wherein the method further comprises transmitting a location of the individual to the provider.

## Description

### Field of the Invention

The invention relates to a method of managing health information.

### Background

This section introduces aspects that may be helpful in facilitating a better understanding of the invention. Accordingly, the statements of this section are to be read in this light and are not to be understood as admissions about what is in the prior art or what is not in the prior art.

Health informatics, also known as health care informatics, healthcare informatics, medical informatics, or biomedical informatics, is a discipline at the intersection of information science, computer science, and health care. It deals with the resources, devices, and methods required in optimizing the acquisition, storage, retrieval, and use of information in health and biomedicine. Health informatics tools include not only computers but also clinical guidelines, formal medical terminologies, and information and communication systems. These are applied to the areas of nursing, clinical care, dentistry, pharmacy, public health, and medical research.

Within the field of health informatics, an electronic health record (EHR), sometimes called an electronic patient record (EPR) or computerized patient record, is an evolving concept defined as a systematic collection of electronic health information about individual patients or populations. It is a record in digital format that is capable of being shared across different health care settings, by being embedded in network-connected enterprise-wide information systems. Such records may include a whole range of data in comprehensive or summary form, including demographics, medical history, medication and allergies, immunization status, laboratory test results, radiology images, vital signs, personal stats like age and weight, and billing information. Their purpose can be understood as a complete documentation of patient encounters that allows the automation and streamlining of the workflow in health care settings and increases safety through evidence-based decision support, quality management, and outcomes reporting.

An EHR that is primarily controlled by the individual patient is commonly labeled a personal health record (PHR). A conventional PHR provides a complete and accurate summary of the health and medical history of an individual by gathering data from many sources and making this information accessible online to anyone who has the necessary electronic credentials. Such data may include information about allergies and adverse drug reactions, medications including over-the-counter medications and herbal remedies, illnesses and hospitalizations, surgeries and other procedures, chronic diseases, vaccinations, laboratory test results, imaging reports such as radiographs, immunization records, family history, and Observations of Daily Living (ODLs). In addition to storing an individual's personal health information, some known PHRs provide added-value services such as drug-drug interaction checking or electronic messaging between patients and providers.

Some advanced PHRs are delivered by means of mobile Smartphone-based applications that may have the capability to print, backup, encrypt, and import data from other sources such as a laboratory, clinic's EHR or Web-based PHR. Health history such as medications, immunizations, and allergies may be stored in the application. One such service is known as World Medical Card and aims at improving the safety for people in a situation requiring immediate medical treatment by a doctor who is not familiar with the person's medical history. In a case of emergency, a medical professional may physically cut open a sealed card, revealing an emergency code allowing access to a read-only web profile describing the card holder. A complementary phone application based on the Wireless Access Protocol (WAP) allows access to the same data by mobile users such as first responders, emergency medical technicians, and paramedics.

### Summary

The present invention was made in view of the prior art described above, and the object of some embodiments is to provide a practical method capable of managing health information.

According to an embodiment there is provided a method of managing health information, the method comprising maintaining a personal health record for an individual by a provider and authorizing access to the personal health record in response to a medical emergency, wherein the method further comprises transmitting a location of the individual to the provider. Here, by medical emergency is meant any injury or illness that is acute and poses an immediate risk to the individual's life or long-term health.

### Description of the Embodiments

Hereinafter, the best mode for carrying out a method according to an embodiment is described in detail.

In this embodiment, the method is applied by a health care provider. In this context, by health care provider is meant a health professional or organization that provides services of a health professional. A health professional in turn is any qualified person who delivers proper health care in a systematic way professionally to any individual in need of a health care service. To gain access to the service described below, the individual may be required to sign up or subscribe to it.

For maximum accessibility, the PHR of this embodiment takes the form of an internet-based or mobile Smartphone-based PHR. To this end, the health care provider maintains the PHR by means of a server remote from the individual instead of on a local machine. Here, by server is meant a software service running on a dedicated computer, configured to serve the needs of the individual through a computer network. More specifically, the server takes the form of a database server, that is, a computer program that provides database services to other computer programs or computers, as defined by the client-server model of computing.

For improved flexibility, the PHR is configurable, such as by means of a Web browser. In particular, in a preliminary step, the individual may choose to define the access rights of other users or application programs. Access rights may also be configured depending on a physiological condition of the individual, such as personal fitness, fatigue, distress, or consciousness.

To allow for remote physiological monitoring of the individual, the latter is equipped with a sensor configured to capture a vital sign, that is, a measure of a physiological statistic permitting to assess one of the most basic body functions of the individual. Here, by sensor is meant any device that measures a physical quantity and converts it into a signal which can be read by an observer or by an instrument. Suitable statistics include the individual's body temperature, pulse rate or heart rate, blood pressure, respiratory rate, and movement. To this end, the individual may be outfitted with a wearable physiological system, for example, a blood pressure cuff, remote sensing waist coat, glucose meter, or solid state accelerometer. For improved comfort, the sensor may optionally be integrated with a smart fabric, sometimes called smart textile.

As an alternative to physiological monitoring of the individual per se, a vehicle or craft operated by the individual may be equipped with an electronic control unit. In automotive electronics, by electronic control unit (ECU), also labeled electronic control module (ECM), central control module (CCM), control unit, or control module, is meant any embedded system that controls one or more of the electrical systems or subsystems of the vehicle or craft. More specifically, the ECU may take the form of an airbag control unit (ACU), that is, an ECU adapted to monitor one or more sensors within the vehicle, such as an accelerometer, impact sensor, side pressure sensor, wheel speed sensor, gyroscope, brake pressure sensor, or seat occupancy sensor.

To detect any medical condition in real time, the results of this physiological or vehicular monitoring are reported to a cellular network transceiver by means of a wireless sensor network (WSN), that is, the sensors involved constitute the nodes of a wireless ad-hoc network where each node is further equipped with a radio transceiver or other wireless communication device, a small microprocessor, and an energy source, usually a battery. The cellular network transceiver may take the form of a mobile phone, also known as a mobile, cellular telephone, cell phone, hand phone, or handy. Alternatively, the transceiver may be integrated with a vehicle-based computer. For maximum control over the entire process, the health care provider may allow to define the triggering event or events for reporting an emergency, such as a collapse of the individual's pulse or severe vehicle impact.

To be able to identify the individual to the health care provider, a unique identification is associated with the former upon subscription to the health care service. For improved interoperability with existing mobile phone hardware, this identification takes the form of an International Mobile Subscriber Identity (IMSI) as is stored in a subscriber identity module or subscriber identification module (SIM) of the mobile phone or vehicle-based computer. In addition to the IMSI, the SIM stores a first authentication key, that is, a 128-bit value used in authenticating the SIM on the mobile network. In the embodiment at hand, an authentication of the SIM to the mobile network provider effectively serves to authenticate the individual to the health care provider. in storing the first authentication key on the SIM, the method according to this embodiment particularly allows for an authentication of the individual without cooperation of the latter, such as in case of a sudden blackout.

Upon detecting an emergency condition such as a loss of consciousness or severe collision, the transceiver, by means of a cellular network, informs the health care provider that a medical emergency is anticipated. In mobile telecommunications, by cellular network is meant a radio network distributed over land areas called cells, each served by at least one fixed-location transceiver known as a cell site or base station. Along with this notification, the transceiver transmits its current time and location to the health care provider. To this end, the transceiver may be integrated with a global navigation satellite system receiver. Here, by global navigation satellite system (GNSS) is meant any satellite navigation (sat nav) system that provides autonomous geo-spatial positioning with global coverage, such as the NAVSTAR Global Positioning System (GPS).

As an alternative to the use of a GNSS, the transceiver's position may be fixed by means of mobile phone tracking, that is, by multilateration (MLAT) based on the signal strength to nearby antenna masts. In yet another embodiment, the health care provider may employ a real-time locating system (RTLS), that is, a system that provides the location of assets on a constant and recurrent basis. For instance, such system may be based on a wireless local area network (WLAN) to which the transceiver connects.

To cater for the case where the individual is physically relocated after the emergency was initially reported, the transceiver may be adapted to transmit periodic location updates in predetermined time intervals, at least until the location is first recalled from the database maintained by the health care provider.

Upon receiving the emergency notification and associated time and location, the health service provider may authenticate the individual by means of the first authentication key described above. To allow a medical professional in charge to make informed treatment choices, the health care provider may further authenticate the medical professional by means of a second authentication key associated with the latter. Having authenticated the individual and attending professional, the health care provider will typically authorize access to the PHR, allowing the medical professional to view the health and medical history of her patient. The provider may further choose to log the time and location of the incident for future use.

For convenient mobile access to the PHR, the medical professional may preferably make use of a portable personal computer (PC) such as a tablet PC, which is equipped with a touch screen as a primary input device and designed to be operated by an end user.

A method according to this embodiment bears the particular benefit of being able to establish the individual's identity in the absence of further credentials. By matching a given location and time to the respective values received along with recent emergency notifications, even an unconscious subject may be identified without further tagging. Such identification may be requested, for instance, by a first aider or emergency physician upon reaching a place of accident based on her own position and current time. This ability is especially advantageous during a state of disaster.

A person of skill in the art would readily recognize that steps of various above-described methods can be performed by programmed computers. Herein, some embodiments are also intended to cover program storage devices, e.g., digital data storage media, which are machine or computer readable and encode machine-executable or computer-executable programs of instructions, wherein said instructions perform some or all of the steps of said above-described methods. The program storage devices may be, e.g., digital memories, magnetic storage media such as a magnetic disks and magnetic tapes, hard drives, or optically readable digital data storage media. The embodiments are also intended to cover computers programmed to perform said steps of the above-described methods.

The description merely illustrates the principles of the invention. It will thus be appreciated that those skilled in the art will be able to devise various arrangements that, although not explicitly described or shown herein, embody the principles of the invention and are included within its spirit and scope. Furthermore, all examples recited herein are principally intended expressly to be only for pedagogical purposes to aid the reader in understanding the principles of the invention and the concepts contributed by the inventor(s) to furthering the art, and are to be construed as being without limitation to such specifically recited examples and conditions. Moreover, all statements herein reciting principles, aspects, and embodiments of the invention, as well as specific examples thereof, are intended to encompass equivalents thereof.

## Claims

1. Method of managing health information, the method comprising maintaining a personal health record for an individual by a provider and authorizing access to the personal health record in response to a medical emergency, wherein the method further comprises transmitting a location of the individual to the provider.

2. Method as in claim 1 wherein the location is transmitted in response to the medical emergency.

3. Method as in claim 1 or 2, further comprising the step of fixing the location by means of satellite navigation, mobile phone tracking, or real-time locating.

4. Method as in any of the preceding claims, further comprising transmitting a time of the medical emergency to the provider.

5. Method as in any of the preceding claims wherein a unique identification is associated with the individual and wherein the method further comprises identifying the individual to the provider.

6. Method as in claim 5 wherein the unique identification is based on an International Mobile Subscriber Identity.

7. Method as in claim 6 wherein a subscriber identity module stores the International Mobile Subscriber Identity.

8. Method as in any of the preceding claims, further comprising authenticating the individual to the provider by means of a first authentication key associated with the individual.

9. Method as in claim 7 and 8 where the subscriber identity module further stores the first authentication key.

10. Method as in claim 8 wherein the personal health record is maintained by means of a server and the first authentication key is maintained by means of a client,

11. Method as in any of claims 8 to 10 wherein the personal health record is accessed by a medical professional and the method comprises the further step of authenticating the medical professional to the provider by means of a second authentication key prior to authorizing access.

12. Method as in any of the preceding claims, further comprising monitoring a vital sign of the individual and detecting the medical emergency based on the vital sign.

13. Method as in any of the preceding claims, further comprising monitoring a condition of a vehicle associated with the individual and detecting the medical emergency based on the condition.

14. Method as in any of the preceding claims comprising the preliminary step of configuring the personal health record by the individual.

15. Method as in claim 14 wherein the configuration of the personal health record comprises defining the rights of further individuals or application programs to access the personal health record.
